# EUROPEAN PATENT SPECIFICATION

(11) **EP 3 003 233 B1**
(45) Date of publication and mention of the grant of the patent: **15.03.2017**
(21) Application number: 14737320.3
(22) Date of filing: 26.05.2014
(51) Int. Cl.: A61F 5/01

(54) **PROPHYLACTIC KNEE BRACE**
PROPHYLAKTISCHE KNIESCHIENE
GENOUILLÈRE PROPHYLACTIQUE

(30) Priority: 28.05.2013 GB 201309515
(43) Date of publication of application: 13.04.2016
(73) Proprietor: Leatt Corporation, Santa Clarita, California 91350 (US)
(72) Inventor: LEATT, Christopher, James, 7550 Durbanville (ZA); DE JONGH, Cornelis, Uys, 7550 Durbanville (ZA); KEEVY, Pieter, Andre, 7550 Durbanville (ZA); STEFFENS, Jörn, Peter, 26135 Oldenburg (DE)
(74) Representative: Sackin, Robert
(86) International application number: PCT/IB2014/061716
(87) International publication number: WO 2014/191895

(56) References cited:
- EP-A1- 0 670 152
- WO-A1-2004/002376
- DE-A1- 19 810 677
- KR-A- 20110 061 193
- US-A1- 2010 331 750

## Description

### FIELD OF THE INVENTION

This invention relates to knee braces for inhibiting the risk of injuries to the knee, particularly, but not exclusively, in motorcycling, skiing and other activities involving similar mechanics.

### BACKGROUND TO THE INVENTION

A device according to the preamble of claim 1 is disclosed in WO 2004/002376 A1. Of the most common causes of knee injuries include: valgus stress/deformation, i.e. outward angulation of the lower leg and foot, which results in medial collateral ligament (MCL) damage and/or damages to meniscuses on lateral side or tibial plateau; and overextension, which results in damage to the anterior cruciate ligament (ACL).

A number of knee braces have been developed to inhibit knee movement of injured knees to restrict knee movement to flexion and extension about a virtual transverse axis and to encourage recovery. These conventional knee braces typically include an upper part that is tightly strapped onto the upper leg well above the knee, a lower part that is tightly strapped onto the lower leg well below the knee, and two hinges that are positioned laterally and medially of the knee on the virtual transverse axis. These knee braces are substantially symmetrical and are configured to avoid applying significant forces on the knee. Some of these knee braces have been adapted to be worn during activities with higher risk of knee injuries, such as off-road motorcycling.

Conventional knee braces have been designed primarily to inhibit knee extension, but the natural flexing movement of the knee is more complex than simple pivotal movement and during natural flexion, the knee does not remain aligned with the transverse axis about which the brace's hinges pivot. One example of the complexity of knee movement beyond simple pivotal movement, is that the tibeal plateau tends to rotate inwards on the femur, during knee extension. This conflict between natural knee flexion and the pivotal movement of conventional knee brace hinges cause discomfort, resistance to knee flexion and wear of the knee braces' hinges.

Some of the other shortfalls experienced when wearing conventional knee braces include: bulk that causes interference with equipment (e.g. that prevents them from being worn inside boots), inadequate engagement with the wearers' thighs and inadequate adjustment to fit different knee and leg shapes.

The present invention seeks to reduce the risk of knee injury, to address the shortfalls of conventional knee braces mentioned above and/or to:
control hyperflexion and hyperextension of the knee and ameliorate overextension;
provide stiffness to protect the knee against side impact and bending of the knee and ameliorate valgus deformation;
allow hinging that does not conflict with natural knee movement;
achieve a stable fit on the wearer's thigh;
achieve a comfortable and effective fit on different knee and leg shapes; and
prolong hinge lifespan.

### SUMMARY OF THE INVENTION

According to the present invention there is provided a protective device according to claim 1.

The hinge mechanism may comprise a link that is pivotally connected to the upper leg formation to pivot relative to the upper leg formation about an upper pivot axis, said link also being pivotally connected to the lower leg formation to pivot relative to the lower leg formation about a lower pivot axis, said upper and lower pivot axes extending generally parallel to each other and extending generally transverse to the wearer's knee.

The hinge mechanism may include an upper rotational element that is fixed relative to the upper leg formation and a lower rotational element that is fixed relative to the lower leg formation and the upper and lower rotational elements may be connected together for counter-rotation. The hinge mechanism may include a flexible tensile element that extends partially around the upper and lower rotational elements and crosses between the upper and lower rotational elements, in a figure-of-eight configuration.

The lower leg formation may comprise a thin plate of stiff material.

The upper leg formation may include elongate tensile elements that extend across the posterior aspect of the wearer's thigh in a crossed configuration and the lower leg formation may include elongate tensile elements that extend across the posterior and lateral aspects of the wearer's calf in a crossed configuration, when the device is worn.

### BRIEF DESCRIPTION OF THE DRAWINGS

For a better understanding of the present invention, and to show how it may be carried into effect, the invention will now be described by way of non-limiting example, with reference to the accompanying drawings in which:
Figure 1 is an anterior view of a first embodiment of a protective device for a left leg of a wearer, in accordance with the present invention;
Figure 2 is an anterior view of a stiff pivotal structure of the protective device of Figure 1;
Figure 3 is an oblique lateral-anterior view of the protective device of Figure 1, with a sock omitted and with a detail sectional view of a hinge mechanism of the protective device;
Figure 4 is a lateral view of the pivotal structure of Figure 2, in flexed and extended conditions;
Figure 5 is a medial view of a second embodiment of a protective device for a left leg of a wearer, in accordance with the present invention (with straps omitted);
Figure 6 is an anterior view of the protective device of Figure 5; and
Figure 7 is lateral view of the protective device of Figure 5.

### DETAILED DESCRIPTION OF THE DRAWINGS

Referring to the drawings, a protective device in the form of a knee brace in accordance with the present invention is generally identified by reference numeral 10. Features that are common between the two illustrated embodiments of the invention are identified by the same reference numerals and suffixes are used to distinguish between different embodiments, where necessary.

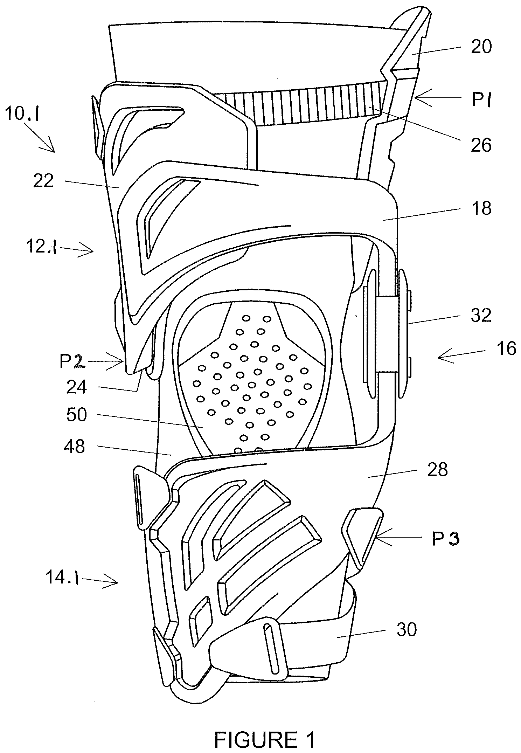

Referring to Figures 1 to 4, the knee brace 10.1.1 includes an upper leg formation 12 that is configured to be attached to a wearer's thigh, a lower leg formation 14 that is configured to be attached to the wearer's calf, and a single, lateral hinge mechanism 16 through which the upper and lower leg formations are pivotally connected.

The upper leg formation 12 includes a frame, which in the illustrated embodiment is a curved frame 18 of a suitably stiff (preferably rigid) material, which extends from the hinge mechanism 16 on the lateral aspect of the knee, over the anterior of the thigh. The upper leg formation 12 further includes an upper lateral pressure formation in the form of a lateral thigh pad 20 that extends along the lateral aspect of the wearer's thigh and presses against the thigh, as well as a medial thigh pad 22 that extends along the medial aspect of the thigh, but that is positioned lower than the lateral thigh pad 20. Each of the thigh pads 20,22 is attached to the frame 18 in a manner that allows forces exerted by the frame to be transferred directly to the thigh pads and to be exerted by the thigh pads as pressure on the wearer's thigh.

The lower end of the medial thigh pad 22 can serve as a medial pressure formation that presses against the medial aspect of the upper leg in the region of the knee, e.g. on the medial aspect of the distal extremity of the femur. However, in the illustrated embodiment, the medial thigh pad 22 includes a medial pressure formation in the form of a protuberance or knee pad 24 that is adjustable to compensate for variations in wearers' knee anatomies and to move the medial pressure formation into contact with the knee complex at the medial aspect of the distal extremity of the femur- referred to herein as the medial pressure point.

The lateral thigh pad 20 and medial thigh pad 22 (with the knee pad 24) are attached to the frame 18 and are held in position on the wearer's thigh, e.g. with elongate tensile elements in the form or straps 26 that extend across the posterior of the thigh in a crossed or other (e.g. circumferential) configuration. The large thigh pads 20,22 distribute pressure over a large area and the crossed configuration of the straps 26 allows the upper leg formation 12 to be attached to the upper leg comfortably, but firmly, while allowing normal muscle action, without undue pressure on the muscles.

The lower leg formation 14 includes a plate 28 of a suitably stiff (preferably rigid) material, which extends from the hinge mechanism 16, along the lateral aspect of the knee and calf, over the anterior of the shin. The plate 28 is wider and quite thin in the shin region and is attached to the wearer's calf by elongate tensile elements in the form or straps 30 that extend across the posterior and lateral aspects of the calf in a crossed or other configuration - which prevents undue pressure on the calf muscles. The thin profile of the plate 28 and straps 30 allow the lower leg formation to be worn with boots. (The "plate" 28 is reasonably thick in the vicinity of the hinge mechanism 16 and does not truly resemble a "plate" in this region, but the name "plate" is used herein to refer to the entire part - for simplicity.)

The straps 26,30 can be tightened with ratchet mechanisms (not shown in the drawings) and can be adjusted easily to ensure that the knee brace 10.1 fits comfortably and tightly on various leg sizes.

The plate 28 forms a lateral pressure formation below its attachment to the hinge mechanism 16, where it presses against the lateral aspect of the calf. The knee brace 10.1 thus includes at least three points where it can exert pressure on the wearer's leg: with the lateral thigh pad 20, knee pad 24 and lateral pressure formation of the plate 28. These three points are shown with arrows on Figure 1, marked "P" (with suffixes), and by exerting pressure on the leg at the three pressure points, the knee brace 10.1 stabilises the knee against valgus stress and thus against meniscus and MCL injury. The pressure points P include a proximal lateral pressure point P1, laterally on the thigh; the medial pressure point P2; and a distal lateral pressure point P3, laterally on the calf.

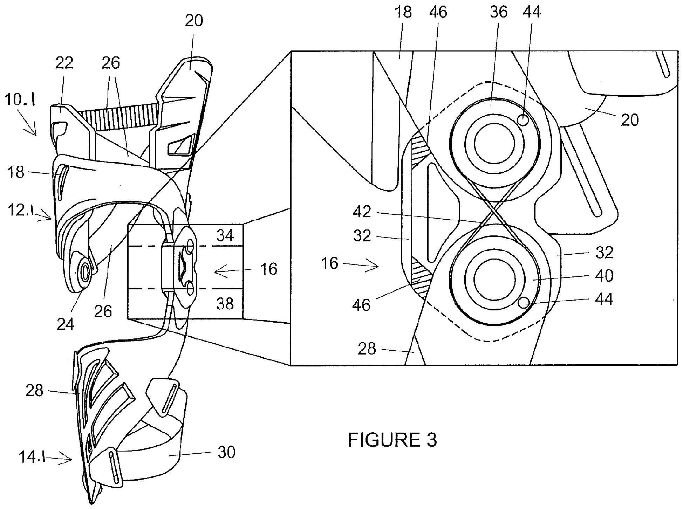

The hinge mechanism 16 includes a housing 32 that forms a link between the upper and lower leg formations 12,14. As shown in Figure 3, a lower end of the frame 18 of the upper leg formation 12 extends inside the housing 32 and is pivotally connected to the housing to rotate relative to the housing about an upper pivot axis 34. An upper rotational element in the form of an upper spool 36 is also supported to rotate about the upper pivot axis 34, with the lower end of the frame 18. Similarly, an upper end of the plate 28 extends inside the housing 32 and is pivotally connected to the housing to rotate relative to the housing about a lower pivot axis 38 and a lower rotational element in the form of a lower spool 40 is supported to rotate about the lower pivot axis, with the upper end of the plate 28. The upper and lower pivot axes 34,38 are generally parallel to each other and extend transverse to the wearer's knee (i.e. extend in a lateral-medial direction).

The upper and lower spools 36,40 are kept a fixed distance apart by the housing 32 and can each rotate relative to the housing, with the upper leg formation 12 and lower leg formation 14, respectively. A flexible tensile element in the form of a steel cable 42 extends partially around the spools 36,40 and crosses between them in a figure-of-eight configuration. The cable 42 is anchored relative to the circumference of each of the spools 36,40 by an anchor element 44, to prevent circumferential slippage of the cable along either spool and as a result, the cable connects the spools for counter rotation relative to the housing. The spools 36,40 and upper and lower leg formations 12,14 are supported for rotation relative to the housing 32 by sealed double bearings - to provide smooth and long lasting pivotal movement.

The double axis pivotal movement in the hinge mechanism 16 replicates human cruciate ligament function and is a much closer simulation to the natural flexion of the wearer's knee, than a simple pivotal movement.

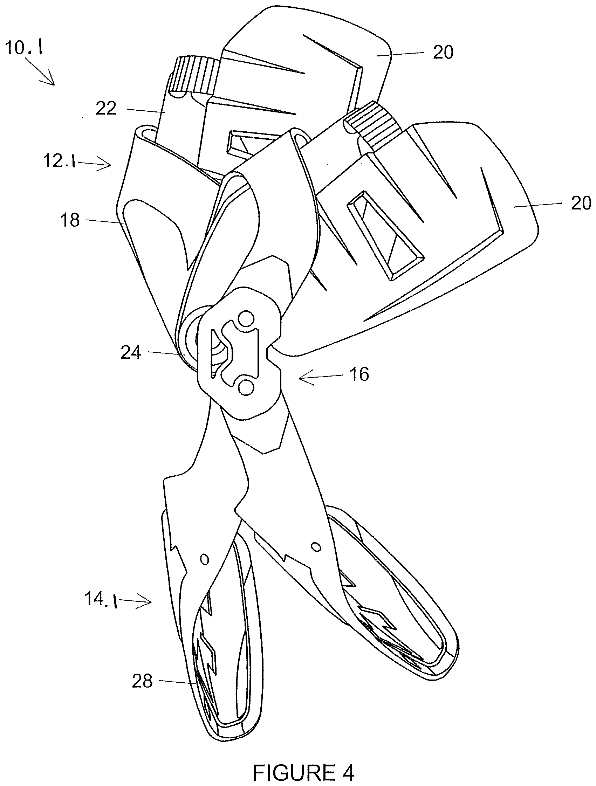

The housing 32 includes stop formations 46 on its anterior side and the stop formations are positioned to contact the front of the lower end of the frame 18 and the front of the upper end of the plate 28, respectively, when the upper leg formation 12 and lower leg formation 14 have pivoted to positions corresponding to maximum permissible knee extension - thus preventing hyperextension (and preventing ACL injury). The stop formations 46 are compressible to provide a soft feel and damping when the knee brace reaches its fully extended position and the positions of the stop formations are adjustable by swapping inserts that form part of the housing 32 (the stop formations 46 forming part of the inserts). The range of pivotal movement that the hinge mechanism 16 allows is shown in Figure 4, where the upper and lower leg formations 12,14 are shown in flexed and maximum extended positions, relative to the housing 32.

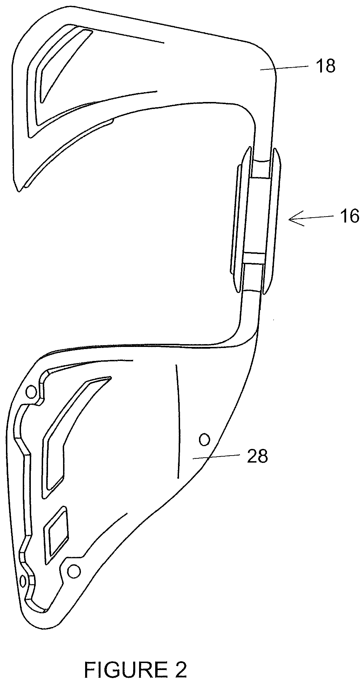

The frame 18, hinge mechanism 16 and plate 28 form a super strong C-shaped structure (shown in Figure 2), but has no medial hinge mechanism, with the result that the knee brace 10.1 is unlikely to interfere other apparatus during use - especially when compared to prior art knee braces with medial hinges that tended to knock against motorcycles while riding. Further, the use of a single lateral hinge and a knee pad 24 that exerts pressure on the wearer's knee complex at the medial pressure point, allows the tibia to rotate when flexing the knee - more particularly: allows inward rotation of the tibial plateau relative to the femur when extending the knee, and vice versa.

The C-shaped structure shown in Figure 2 (which includes the plate 28), together with the lateral thigh pad, the lower part of the medial thigh pad 22, and the knee pad 24, form a strong, stiff structure, applying pressure on the three pressure points P1-P3 that stabilise the knee against valgus stress and thus against meniscus and MCL injury. The remainder of formations that can exert pressure on the thigh and calf, do so merely to the extent required to hold the knee brace 10.1 in place - in similar fashion to the attachment of prior art knee pads to wearers' legs.

The knee brace 10.1 preferably includes an integrating sock 48 with a visco-elastic patella cup 50, to provide impact protection and a good fit of the knee brace on the wearer's leg.

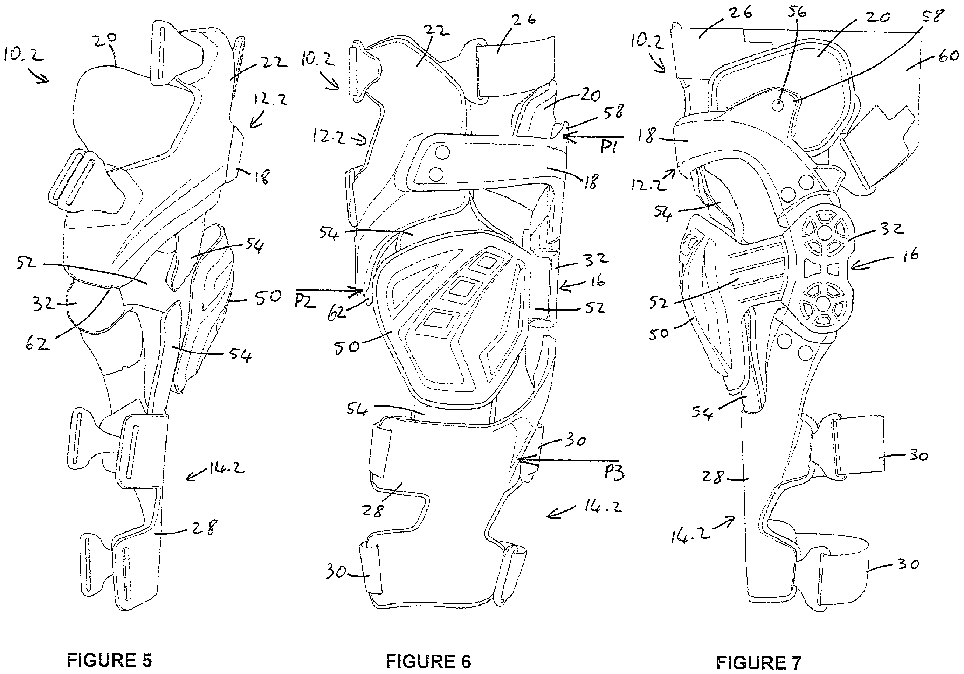

Referring to Figures 5 to 7, a second embodiment of a knee brace 10.2 according to the present invention, is substantially similar to the first embodiment shown in Figures 1 to 4, apart from the following differences.

The patella cup 50 is not held in position by a sock, but is held in position by a rigid support arm 52 extending between the housing 32 of the hinge mechanism 16 and the patella cup. Additional protecting plates 54 extend down from the medial thigh pad 22 and up from the plate 28 partly behind the patella cup 50.

The lateral thigh pad 20 is attached to a rigid tab 58 that forms part of the frame 18, by a pivotal pin 56 and this allows the thigh pad 20 to be pivoted and/or rotated about the pin to provide comfort and good load distribution for pressure exerted between the lateral thigh and the frame, at the proximal lateral pressure point P1.

The medial pressure formation of the knee brace 10.2 is not an adjustable knee pad, but instead, the distal end 62 of the medial thigh pad 22 forms a medial pressure formation and presses on the knee complex at the medial pressure point P2.

The plate 28 extends farther posterior in the region of the distal lateral pressure point P3.

The straps 26 holding the upper leg formation in place, are partly substituted and/or supplemented with webbing 60 (shown in Figure 7) in the form of stretchable fabric that can extend around part of the thigh.

## Claims

1. A protective device (10) which includes:
an upper leg formation (12) that is attachable to the upper leg of a wearer and that includes an upper lateral pressure formation (20) that presses against the lateral aspect of the wearer's upper leg at a proximal lateral pressure point (P1), when the device (10) is worn;
a lower leg formation (14) that is attachable to the lower leg of the same leg of the wearer and that includes a lower lateral pressure formation (18) that presses against the lateral aspect of the wearer's lower leg, at a distal lateral pressure point (P3), when the device (10) is worn;
a hinge mechanism (16) that is connected to the upper leg formation (12) and to the lower leg formation (14), said hinge mechanism (16) being configured to allow pivotal movement between the upper leg formation (12) and the lower leg formation (14) about at least one pivot axis (34,38) that extends generally transverse to the wearer's knee, said hinge mechanism (16) being disposed entirely on the lateral side of the wearer's knee, when the device (10) is worn; and
a medial pressure formation (24,62) that forms part of the upper leg formation (12) and that presses against the knee complex at a medial pressure point (P2) on the medial aspect of the distal extremity of the femur, when the device (10) is worn;
**characterised in that** said upper lateral pressure formation (20), medial pressure formation (24,62) and lower lateral pressure formation (28) form a strong, stiff structure which includes the hinge mechanism (16), said stiff structure applying pressure on the proximal lateral pressure point (P1), the distal lateral pressure point (P3) and the medial pressure point (P2) that stabilise the knee against valgus stress.

2. A protective device (10) according to claim 1, wherein the hinge mechanism (16) comprises a link (32) that is pivotally connected to the upper leg formation (12) to pivot relative to the upper leg formation (12) about an upper pivot axis (34), said link (32) also being pivotally connected to the lower leg formation (14) to pivot relative to the lower leg formation (14) about a lower pivot axis (38), said upper and lower pivot axes (34,38) extending generally parallel to each other and extending generally transverse to the wearer's knee, when the device (10) is worn.

3. A protective device (10) according to claim 1 or claim 2, wherein the hinge mechanism (16) includes an upper rotational element (36) that is fixed relative to the upper leg formation (12) and a lower rotational element (40) that is fixed relative to the lower leg formation (14) and the upper and lower rotational elements (36,40) are connected together for counter-rotation.

4. A protective device (10) according to claim 3, wherein the hinge mechanism (16) includes a flexible tensile element (42) that extends partially around the upper and lower rotational elements (36,40) and crosses between the upper and lower rotational elements (36,40), in a figure-of-eight configuration.

5. A protective device (10) according to any one of the preceding claims,
wherein the lower leg formation (14) comprises a thin plate (18) of stiff material.

6. A protective device (10) according to any one of the preceding claims,
wherein the upper leg formation (12) includes elongate tensile elements (26) that extend across the posterior aspect of the wearer's thigh in a crossed configuration, when the device (10) is worn.

7. A protective device (10) according to any one of the preceding claims,
wherein the lower leg formation (14) includes elongate tensile elements (30) that extend across the posterior and lateral aspects of the wearer's calf in a crossed configuration, when the device (10) is worn.

## Patentansprüche

1. Schutzvorrichtung (10) mit:
einem Oberschenkelgebilde (12), das am Oberschenkel eines Trägers anbringbar ist und das ein oberes Lateraldruckgebilde (20) beinhaltet, das an einem proximalen Lateraldruckpunkt(P1) gegen die laterale Seite des Oberschenkels des Trägers presst, wenn die Vorrichtung (10) getragen wird;
einem Unterschenkelgebilde (14), das am Unterschenkel desselben Beins des Trägers anbringbar ist und das ein unteres Lateraldruckgebilde (18) beinhaltet, das an einem distalen Lateraldruckpunkt (P3) gegen die laterale Seite des Unterschenkels des Trägers presst, wenn die Vorrichtung (10) getragen wird;
einem Gelenkmechanismus (16), der mit dem Oberschenkelgebilde (12) und mit dem Unterschenkelgebilde (14) verbunden ist, wobei der genannte Gelenkmechanismus (16) gestaltet ist, um Schwenkbewegungen zwischen dem Oberschenkelgebilde (12) und dem Unterschenkelgebilde (14) um wenigstens eine Schwenkachse (34, 38), die zum Knie des Trägers allgemein quer verläuft, zuzulassen, wobei der genannte Gelenkmechanismus (16) vollkommen auf der lateralen Seite des Knies des Trägers angeordnet ist, wenn die Vorrichtung (10) getragen wird; und
einem Medialdruckgebilde (24, 62), das Teil des Oberschenkelgebildes (12) bildet und das an einem Medialdruckpunkt (P2) an der medialen Seite des distalen Endes des Femurs gegen den Kniekomplex presst, wenn die Vorrichtung (10) getragen wird;
**dadurch gekennzeichnet, dass** das genannte obere Lateraldruckgebilde (20), Medialdruckgebilde (24, 62) und untere Lateraldruckgebilde (28) eine starke, steife Konstruktion bilden, die den Gelenkmechanismus (16) beinhaltet, wobei die genannte steife Konstruktion Druck auf den proximalen Lateraldruckpunkt (P1), den distalen Lateraldruckpunkt (P3) und den Medialdruckpunkt (P2) ausübt, die das Knie gegen Valgusstress stabilisieren.

2. Schutzvorrichtung (10) nach Anspruch 1, wobei der Gelenkmechanismus (16) ein Verbindungsglied (32) aufweist, das schwenkbar mit dem Oberschenkelgebilde (12) verbunden ist, um relativ zum Oberschenkelgebilde (12) um eine obere Schwenkachse (34) zu schwenken, wobei das genannte Verbindungsglied (32) auch schwenkbar mit dem Unterschenkelgebilde (14) verbunden ist, um relativ zum Unterschenkelgebilde (14) um eine untere Schwenkachse (38) zu schwenken, wobei die genannte obere und untere Schwenkachse (34, 38) allgemein parallel zueinander verlaufen und allgemein quer zum Knie des Trägers verlaufen, wenn die Vorrichtung (10) getragen wird.

3. Schutzvorrichtung (10) nach Anspruch 1 oder Anspruch 2, wobei der Gelenkmechanismus (16) ein oberes drehfähiges Element (36), das relativ zu dem Oberschenkelgebilde (12) fest ist, und ein unteres drehfähiges Element (40), das relativ zum Unterschenkelgebilde (14) fest ist, beinhaltet und das obere und das untere drehfähige Element (36, 40) gegenläufig miteinander verbunden sind.

4. Schutzvorrichtung (10) nach Anspruch 3, wobei der Gelenkmechanismus (16) ein flexibles Spannelement (42) beinhaltet, das teilweise um das obere und das untere drehfähige Element (36, 40) verläuft und sich zwischen dem oberen und dem unteren drehfähigen Element (36, 40) in einer Achterform überkreuzt.

5. Schutzvorrichtung (10) nach einem der vorhergehenden Ansprüche, wobei das Unterschenkelgebilde (14) eine dünne Platte (18) aus steifem Material aufweist.

6. Schutzvorrichtung (10) nach einem der vorhergehenden Ansprüche, wobei das Oberschenkelgebilde (12) längliche Spannelemente (26) beinhaltet, die in einer gekreuzten Form über die posteriore Seite des Schenkels des Trägers verlaufen, wenn die Vorrichtung (10) getragen wird.

7. Schutzvorrichtung (10) nach einem der vorhergehenden Ansprüche, wobei das Unterschenkelgebilde (14) längliche Spannelemente (30) beinhaltet, die in einer gekreuzten Form über die posteriore und die laterale Seite der Wade des Trägers verlaufen, wenn die Vorrichtung (10) getragen wird.

## Revendications

1. Dispositif de protection (10) qui comporte :
une formation de jambe supérieure (12) destinée à être attachée à la jambe supérieure d'un porteur et comportant une formation de pression latérale supérieure (20) qui presse contre l'aspect latéral de la jambe supérieure du porteur à un point de pression latéral proximal (P1), quand le dispositif (10) est porté ;
une formation de jambe inférieure (14) destinée à être attachée à la jambe inférieure de la même jambe du porteur et comportant une formation de pression latérale inférieure (18) qui presse contre l'aspect latéral de la jambe inférieure du porteur à un point de pression latéral distal (P3), quand le dispositif (10) est porté ;
un mécanisme d'articulation (16) qui est raccordé à la formation de jambe supérieure (12) et à la formation de jambe inférieure (14), ledit mécanisme d'articulation (16) étant configuré pour permettre un mouvement pivotant entre la formation de jambe supérieure (12) et la formation de jambe inférieure (14) autour d'au moins un axe pivot (34,38) qui s'étend généralement transversalement au genou du porteur, ledit mécanisme d'articulation (16) étant disposé entièrement sur le côté latéral du genou du porteur, quand le dispositif (10) est porté ; et
une formation de pression médiale (24,62) qui fait partie de la formation de jambe supérieure (12) et qui presse contre le complexe du genou à un point de pression médial (P2) sur l'aspect médial de l'extrémité distale du fémur, quand le dispositif (10) est porté ;
**caractérisé en ce que** ladite formation de pression latérale supérieure (20), la formation de pression médiale (24,62) et la formation de pression latérale inférieure (28) forment une structure solide, rigide qui comporte le mécanisme d'articulation (16), ladite structure rigide appliquant une pression sur le point de pression latéral proximal (P1), le point de pression latéral distal (P3) et le point de pression médial (P2) qui stabilise le genou contre une tension en valgus.

2. Dispositif de protection (10) selon la revendication 1, dans lequel le mécanisme d'articulation (16) comprend une liaison (32) qui est raccordée de manière pivotante à la formation de jambe supérieure (12) pour pivoter par rapport à la formation de jambe supérieure (12) autour d'un axe pivot supérieur (34), ladite liaison (32) étant également raccordée de manière pivotante à la formation de jambe inférieure (14) pour pivoter par rapport à la formation de jambe inférieure (14) autour d'un axe pivot inférieur (38), lesdits axes pivot supérieur et inférieur (34,38) s'étendant généralement parallèlement l'un à l'autre et s'étendant généralement transversalement au genou du porteur, quand le dispositif (10) est porté.

3. Dispositif de protection (10) selon la revendication 1 ou la revendication 2, dans lequel le mécanisme d'articulation (16) comporte un élément de rotation supérieur (36) qui est fixé par rapport à la formation de jambe supérieure (12) et un élément de rotation inférieur (40) qui est fixé par rapport à la formation de jambe inférieure (14) et les éléments de rotation supérieur et inférieur (36,40) sont raccordés ensemble en vue d'une contre-rotation.

4. Dispositif de protection (10) selon la revendication 3, dans lequel le mécanisme d'articulation (16) comporte un élément de tension flexible (42) qui s'étend partiellement autour des éléments de rotation supérieur et inférieur (36,40) et se croise entre les éléments de rotation supérieur et inférieur (36,40) en une configuration en huit.

5. Dispositif de protection (10) selon l'une quelconque des revendications précédentes, dans lequel la formation de jambe inférieure (14) comprend une mince plaque (18) d'un matériau rigide.

6. Dispositif de protection (10) selon l'une quelconque des revendications précédentes, dans lequel la formation de jambe supérieure (12) comprend des éléments de traction allongés (26) qui s'étendent en travers de l'aspect postérieur de la cuisse du porteur en une configuration croisée, quand le dispositif (10) est porté.

7. Dispositif de protection (10) selon l'une quelconque des revendications précédentes, dans lequel la formation de jambe inférieure (14) comprend des éléments de traction allongés (30) qui s'étendent en travers des aspects postérieur et latéral du mollet du porteur en une configuration croisée, quand le dispositif (10) est porté.
